Europäisches Patentamt

European Patent Office

Office européen des brevets

(19)

(11) Publication number: **0 103 081 B1**

# EUROPEAN PATENT SPECIFICATION

(45) Date of publication of patent specification: **04.12.91**

(51) Int. Cl.⁵: **A61D 7/00, A61M 25/00, A61M 1/00**

(21) Application number: **83105982.9**

(22) Date of filing: **18.06.83**

(54) Method of growing cells in vitro.

(30) Priority: **30.03.83 US 480355**
**06.07.82 US 395830**

(43) Date of publication of application:
**21.03.84 Bulletin 84/12**

(45) Publication of the grant of the patent:
**04.12.91 Bulletin 91/49**

(84) Designated Contracting States:
**AT BE CH DE FR GB IT LI LU NL SE**

(56) References cited:
**EP-A- 0 022 370          CH-A- 305 264**
**FR-A- 2 496 466          GB-A- 1 154 623**
**US-A- 3 401 689          US-A- 3 540 451**
**US-A- 3 570 484          US-A- 3 663 965**
**US-A- 4 315 513          US-A- 4 344 435**

(73) Proprietor: **HTI BIO-PRODUCTS, INC.**
**P.O. Box 1319**
**Ramona, California 92065(US)**

(72) Inventor: **Cullor, James S.**
**128 Shasta Place**
**Woodland Calif. 95615(US)**

(74) Representative: **UEXKÜLL & STOLBERG Patentanwälte**
**Beselerstrasse 4**
**W-2000 Hamburg 52(DE)**

**Description**

The invention relates to a method of growing cells in vitro in a medium containing allantoic fluid of a pregnant animal having a placenta in combination with bovine calf serum. In preferred methods, monoclonal antibody production is enhanced by in vitro education of the cells using allantoic fluid; moreover, use of the allantioc fluid of a pregnant host (e.g. cow, horse, sheep or pig) after the onset of fetus immumocompetency is especially advantageous.

When a foreign substance enters the body of a vertebrate animal or is injected into it, one aspect of the immune response is the secretion by plasma cells of antibodies. Quite apart from the natural function of the antibodies in the animal's immune response, such antibodies have long been an important tool for investigators, who capitalize on their specificity to indentify or label particular molecules or cells and to separate them from a mixture. The antibody response to a typical antigen is normally highly heterogeneous, and even the best of antisera are really heterogeneous mixtures of many different antibody molecules that vary in charge, size, and in such biologic properties as the ability to fix complement or to agglutinate or precipitate antigen. It is extremely difficult to separate the various antibodies in antisera, and therefore conventional antisera contain mixtures of antibodies, and such mixtures vary from animal to animal.

It is also known that malignant tumors of the immune system (called myelomas) are characterized by rapidly proliferating cells producing large amount of abnormal immunoglobulines called myeloma proteins. A tumor itself is considered to be an immortal clone of cells descended from a single progentior, and so myeloma cells can be cultured indefinitely, and all the immunoglobulins they secrete are identical in chemical structure. They are in effect monoclonal antibodies, but there is no way to know what antigen they are directed against, nor can one induce myelomas that produce antibody to a specific antigen. However, in recent years researchers have learned how to fuse myeloma cells of mice with lymphocytes from the spleen. of mice immunized with a particular antigen. The resulting hybrid-myeloma, or "hybridoma" cells express both the lymphocyte's property of specific antibody production and the immortal character of the myeloma cells. Such hybrid cells can be manipulated by the techniques applicable to animal cells in permanent culture. Individual hybrid cells can be cloned, and each clone produces large amounts of identical antibody to the single antigenic determinant. The individual clones can be maintained indefinitely, and at any time samples can be grown in culture or injected into animals for large scale production of monoclonal antibody. Highly specific monoclonal antibodies produced by this general method have proved to be a versatile tool in many areas of biological research and clinical medicine.

While the utility of specific monoclonal antibodies is manifest, a problem has arisen because of the difficulty of producing significant (e.g., liter) quantities of the antibodies. Obviously, the production of such antibodies in mice is not at all suited for large scale production.

In response to this problem, it has been suggested to employ large mammals such as cattle or sheep for in vivo production of monoclonal antibodies. However , the prior practice of simply placing a cell line in amniotic fluid often leads to rapid death of a large proportion of cells. That is to say, cell lines of interest are typically cultured in highly specific and optimized media, and under relative critical conditions. For example, many cell lines are cultured and allowed to multiply at incubation temperatures of 37 degrees centigrade in specially prepared media, with periodic screening and feeding at regular intervals (e.g., every 48-72 hours). Prior to inoculation, the cells are concentrated into a fixed volume of their media plus fetal calf serum and are surgically implanted. However, this procedure gives only mediocre results, and can often fail completely in that the cells do not multiply or do not produce the desired antibody. One problem with this approach is that the cells, when inoculated, can experience severe "shock" owing to the radically different ambient environment of the amniotic fluid as compared with the previous in vitro culture media and scrupulously maintained growth conditions.

According to the invention this problem is solved by "educating" cells prior to inoculation therof into the host organ. Such education includes growing cells in vitro in a medium containing allantoic fluid of a pregnant animal having a placenta, comprising removing a quantity of allantoic fluid from the animal, mixing an amount of bovine calf serum with said combined product in an in vitro vessel separate and remote from said animal, placing cells to be grown in contact with said combined product in said vessel, and allowing said cells to grow therein.

An in vitro method of growing cells, wherein the allantoic fluid of a pregnant Placentalia is used as a culture medium is described in FR-A-2 496 466. However, this publication refers to a very specific cell type, namely HLPh-producing cells and it does not give any indication of the neccessity or advantage of using bovine calf serum as further supplement.

Typically, according to the invention the cells are repeatedly contacted with respective quantities of the allantoic fluid over a period of time, with the concentration of the allantoic fluid being increased. In the case of bovine allantoic fluid, the initial contact would be with a fluid containing from about 5 to 10 percent by volume of the allantoic fluid, followed by step-wise contacting of the cells with additional fluid samples containing increasing concentrations of the allantoic fluid. During this education procedure, the cells become acclimated to the allantoic fluid and begin to multiply therein.

In a typical procedure for the production of monoclonal antibodies, the cow's uterus or fetus is inoculated. Such inoculation would include introduction of conditioning reagents (e.g. pristane, albumins and the like) in uterine fluids, followed by inoculation of the cell lines. Incubation varies with the specific cell line selected. Typical enrichment constituents added to the uterine fluid during the incubation sequence would include, amino acids, bovine serum albumin, vitamins, inorganic salts, and suspension mediums and growth factors. More specifically, amino acids such as 1-glutamine, 1-argine, 1-cystine, and 1-histadine HCL.$H_2O$ may be added. Vitamin addition may include d-calcium pantothenate, thiamine HCL, choline chloride and riboflavin. Inorganic salts may include KCL, $NaHCO_3$, $NaH_2PO_4.H_2O$, and $CaCL_2.2H_2O$. Finally, suspension mediums such as dextrose, phenol red, $MgCL_2.6H_2O$, and NaCL, KCL may be included.

As noted above, the method of the invention is particularly adapted for the preparation of large-scale in vivo production of monoclonal antibodies. To this end, it has been discovered that the cell line to be employed should be initially educated to the anticipated in vivo environment. Such education generally refers to an adjustment of the growth and metabolism characteristics of the cells to the new environment, and is generally accomplished by an in vitro contact of the cells with the mixture of allantoic fluid and bovine calf serum.

In practice, the cell education technique normally involves repeated or stepwise contact of the cells with respective, increasing quantities of the mixture of allantoic fluid and bovine calf serum over a period of time, typically as long as several days to several weeks. The allantoic fluid can be derived from the host itself, or more usually from the same type of animal of the host.

In particularly preferred forms, the host animal is a pregnant cow. In this case, the cells are contacted with a mixture of various nutrients and the mixture of an allantoic fluid and bovine calf serum until cell acclimation is established by the appropriate in vitro growth rate. During Such in vitro cell education, the cells are advantageously maintained at a temperature of from about 38.3 - 39.0 degrees centrigrade. In addition, the cells are initially contacted with a fluid containing from about 5 to 10 percent by volume of the allantoic fluid, followed by cultivation and incubation therein. Thereafter, the cells may be contacted with a fluid in increasing concentrations to achieve optimal growth and education.

EXAMPLES

The following examples described techniques in accordance with the invention. It is to be understood, however, that nothing on the examples should be taken as a limitation on the overall scope of the invention. Rather, the examples are for illustrative purposes only, in order to eluciate the principles of the invention.

EXAMPLE I

This example sets forth a procedure for the in vitro education of hybridoma cells. The materials employed were:

POI-STOCK

| Oxalacetic acid | 660 mg. |
|---|---|
| Pyruvate | 250 mg. |
| Distilled $H_2O$ | 50 ml. |
| Insulin | 40 mg. |

The above materials are slightly heated while stirring, and are aliquoted and frozen.

HT-Stock

3

| | |
|---|---|
| Hypoxanthine | 136 mg. |
| Thymidine | 38.8 mg. |
| Distilled $H_2O$ | 100 ml. |

The above materials are mixed, aliquoted and frozen.

H-T Medium

| | |
|---|---|
| RPMI | 325 ml. |
| NCTC-135 | 50 ml. |
| Fetal Calf Serum (FCS) | 100 ML. |
| POI-Stock | 5 ml. |
| HT-Stock | 5 ml |
| L-Glutamin | 5 ml. |
| Pen/Strep | 5 ml. |
| Non-essential Amino Acids | 5 ml. |

The RPMI medium is commercially available (e.g., Flow Laboratories, Cat. No. 12-603) and contains inorganic salts, amino acids, vitamins and other components. Similarly, the NCTC-135 medium is available from Catalog No. 44-1100 (1980) of Gibco Laboratories, 519 Aldo Avenue, Santa Clara, California 95050. The components of the NCTC-135 medium are: inorganic salts (e.g., $CaCl_2$), amino acids (e.g., glycine), vitamins (e.g., niacin), co-enzymes (e.g., FAD, flavin, adenine, dinucleotide), reducing agent (e.g., ascorbic acid), nucleic acid derivative (e.g., thymidine) and additional components (e.g., d-glucose).

The Pen/Strep is a mixture of penicillin and streptomycin, and is available from a number of sources, including the Pfizer Chemical Co. The product contains 5,000 I.U./ml. penicillin and 5000 mcg./ml. streptomycin.

Amniotic Fluid

Obtained from cattle slaughterhouse from pregnant cow at not more than 80 days gestation. The fluid is filtered through Seitz filters (several steps) beginning with a pore size of 1.0 m, and decreasing pore size in stages (0.5, 0.2 and 0.1 mm). The filtered fluid is then heated to 56 degrees centigrade for30 minutes. The fluid may then be tested for bovine virus diarrhea, bluetongue, lepto-spirosis, mycoplasma, or any other agents deemed necessary by the investigation laboratory, for example BVD, lepto, bluetongue, the presence of endotoxin, bacteria, etc.

Heat Inactivated FCS

FCS is heated to 56 degrees C for 30 minutes, and is filtered before use using staged Seitz filters having pore sizes of 0.5 and 0.1 m.

In a specific cell education procedure, the cells (produced by fusion of NS-1 myeloma cells with mouse spleen cells immunized against IBR in cattle) were initially cultured at 38.3-39 degrees centigrade in H-T medium to a density of $10^5$ cells/ml. Ten percent heat inactivated FCS and five percent amniotic fluid were then added, and the mixture was allowed to incubate at 38.3-39 degrees centigrade for 48 hours. At the end of this period, another 5% amniotic fluid was added, and incubation was allowed to continue at the noted temperature.

When the cells grew to a density of $10^6$ cells/ml., the cells were split 1:3 using H-T medium with ten percent heat inactivated FCS and twenty percent amniotic fluid. The cells were then allowed to grow to $10^6$ cells/ml. density at 38.3-39 degrees centigrade, and were again split using the same procedure but with thirty percent amniotic fluid. Finally, all of the resultant cell colonies were grown to a density of $10^6$

cells/ml., and were checked for antibody production.

The entire cell education procedure took four days.

EXAMPLE II

In this in vitro test, hybridoma cells as used in Example 1 were employed.

In a control, the cells were removed from their standard media and placed in allantoic fluid.

A death rate of 90-100 percent occurred within 24 hours of incubation at 39 degrees centigrade. No cells were found alive or producing antibody after 72 hours.

In a second test, the protocol above was generally followed except that the cells and their standard culture media (RPMI) was added to the allantoic fluid (resultant media was 70 percent RPMI, 30 percent allantoic fluid). The initial death rate was decreased to 60 percent after 24 hours of incubation at 39 degrees centigrade. The cells continued to multiply in vitro with the 70 percent RPMI/30 percent uterine fluid media during standard hybridoma culture and feeding techniques.

The second test was repeated except that FCS was added stepwise in various concentrations (5%; 10%; 15%; and 20%). These combinations were successful in lowering the initial death rate to 45-50 percent under standard in vitro culture methods at 39 degrees centigrade. Over several weeks of culturing, the proportion of RPMI was gradually reduced to less than 10 percent. At this point the hybridomas were educated, and were placed in the appropriate (90% allantoic fluid/10% FCS) fluid (in vitro) for multiplication.

Next the cells were fed 10 percent FCS by volume every forty-eight hours for six feedings. At this point the cells continued to grow (multiply) at their normal rate plus maintained their antibody production level, even though subsequent feedings were discontinued. The firt decline in multiplication rate was observed twelve days after the last feeding. Normally, hybridomas must be fed every forty-eight hours; however, the described education to the allantoic fluid allowed this time period to be extended.

EXAMPLE III

This example gives a procedure for the education of myeloma or hybridoma cells prior to inoculation into the allantoic fluid of a pregnant cow.

Materials

1. Allantoic Fluid

Obtained from cattle slaughterhouse from pregnant cows at 3-9 months gestation. The fluid is clarified by low speed centrifugation and sterilized by filtration successively through a series of filters from 1.0 m down to 0.2 m. Endotoxin-free samples of fluid are pooled for use in tissue culture.

2. Media

```
              Dulbecco's Minimal
              Essential Medium           40-75%

              Fetal calf serum           20

              Pen/Strep, 10,000 units/
              10,000 mcg                 1

                                  1
              Nutrient supplement        4

              Allantoic fluid            0-40

      1
       Nutrient supplement
              L-glutamine, 200,mM, 100X

              MEM Amino Acids, 100X

              MEM Non-essential Amino
                      Acids, 100X

              Vitamins, 100X
```

## Method

In a control test, myeloma cells capable of producing antibody against surface antigens of pneumococcus organism were placed in allantoic fluid and tested for survival over a period of 72 hours. A death rate of 50% occurred in 24 hours and at 72 hours 10 percent of the cells were viable.

These myeloma cells were used in an in vitro education test to allantoic fluid. Cells were seeded at $5 \times 10^5$/ml in complete medium. Subsequent passage was set up with the media supplemented by 10% every 48 hours during feeding, up to a maximum level 40 percent allantoic fluid. During this test the growth rate of the myeloma cells remained steady up to the 40 percent allantoic fluid level.

## EXAMPLE IV

The cell line chosen for this study was the murine BALB/C myeloma MOPC 315J which constitutively produces a monoclonal IGA molecule having binding specificity for the trinitrophenol group (TNP). 315J cells are grown in Dulbecco's minimal essential medium supplemented with 20 percent fetal calf serum, antibiotics and a nutrient cocktail consisting of L-glutamine, MEM amino acids, MEM non-essential amino acids, sodium pyruvate, and vitamins. Supernatants of 315J cells grown for a period of time routinely contain up to 1 microgram/ml IgA as measured by radio-immunoassay (RIA). Cultures are routinely grown from $5 \times 10^5$ cells/ml to a concentration 2-5 $\times 10^6$ cells per ml. with refeeding every 2 days. Frozen stocks are maintained at -70 degrees centigrade or in liquid nitrogen.

The antibody production by 315 J cells is routinely measured by RIA or any enzyme-linked immunoadsorbant assay (ELISA). These assays will quantitate the concentration of antibody in either culture fluids or uterine fluids. The rosette assay will identify and quantitate the number of 315J myeloma cells in culture or from uterine fluids. This assay involves attaching the binding antigen (TNP) to sheep red blood cells. Using these assays (RIA, ELISA AND Rosette) both myeloma (hybridoma) cells and their products, monoclonal antibodies, can be identified and quantitated.

Respective colonies of cells were placed in allantoic fluid, in the usual complete medium, and in balanced salt solution and incubated in vitro. Tests were made for cell survival over a period of 72 hours. A death rate of fifty percent occurred in 24 hours and at 72 hours 10 percent of the myeloma cells were viable when they were in allantoic fluid. On the other hand, the cells in balanced salt solution died at a faster rate, 50 percent in 6 hours and 100 percent in 20 hours. Myeloma cells cultured in complete media doubled in number by 24 hours. Those results demonstrated that the allantoic fluid is not toxic but does not have enough nutrients to induce these cells to divide.

The next step was to educate the myeloma cells to grow in medium containing allantoic fluid. Cells were seeded at 5 x $10^5$/ml in complete medium. Subsequent passage was set up with the media supplemented with 10 percent allantoic fluid. The percentage of allantoic fluid was increased by 10 percent with every 48 hours feeding up to 40 percent allantoic fluid. By this method the growth rate of the myeloma cells remained steady.

Myeloma cells, both educated and uneducated, were then injected in the allantoic fluid of pregnant sheep at approximately 100 days gestation, after the onset of fetal immunocompetency. The device employed for the cell inoculation and subsequent fluid withdrawals was of the type illustrated in Figs. 10-11, and the device was installed in the manner described previously, where the inner end of the fluid conduit was affixed to the allantoic sac of the sheep. Pathological samples of tissue from the sheep indicated that myeloma-like dells are found in groups in the placental tissue in cases of both educated and uneducated myeloma cell injection.

Cells taken from samples of sheep allantoic fluid were also counted with time after injection. In one example, $10^8$ uneducated myeloma cells were injected into a sheep with 500 ml. allantoic fluid (estimated). Ten ml. samples were removed daily for 7 days. Beginning with day 1, 8.5 x $10^7$ cells were estimated to be in the allantoic fluid. Cell numbers decreased daily but by 7 days 2.5 x $10^7$ cells were still present. This is a tremendous number of cells to be left when the cells injected were uneducated and no nutrients were added for their growth.

## Claims

1. A method of growing cells in vitro in a medium containing allantoic fluid of a pregnant animal having a placenta, comprising removing a quantity of allantoic fluid from the animal, mixing an amount of bovine calf serum with said allantoic fluid to form a combined product, placing said combined product in an in vitro vessel separate and remote from said animal, placing cells to be grown in contact with said combined product in said vessel, and allowing said cells to grow therein.

2. The method of claim 1. wherein said animal is selected from the group consisting of sheep, horses, cows and pigs.

3. The method of claims 1 or 2, wherein said cells are hybridoma cells.

4. The method of claims 1 to 3, wherein said bovine calf serum comprises fetal calf serum.

5. A culture medium for in vitro growth of cells containing a quantity of allantoic fluid of a pregnant animal having a placenta, characterized in that it further contains an amount of of bovine calf serum mixed with the same.

6. The medium of claim 5, said bovine calf serum comprising fetal calf serum.

7. The medium of claims 5 or 6, wherein said animal is selected from the group consisting of sheep, horses, cows and pigs.

## Revendications

1. Procédé de culture de cellules in vitro dans un milieu contenant du liquide allantoïdien d'un animal gravide ayant un placenta, comprenant le prélèvement d'une quantité de liquide allantoïdien sur l'animal, le mélange d'une quantité de sérum de veau bovin audit liquide allantoïdien pour former un produit combiné, l'introduction dudit produit combiné dans un récipient in vitro séparé et éloigné dudit animal, la mise en contact des cellules à cultiver avec ledit produit combiné dans ledit récipient et la culture desdites cellules dans celui-ci.

2. Procédé selon la revendication 1, dans lequel ledit animal est sélectionné dans le groupe comprenant les brebis, les juments, les vaches et les truies.

3. Procédé selon les revendications 1 ou 2, dans lequel lesdites cellules sont des cellules d'hybridome.

4. Procédé selon les revendications 1 à 3, dans lequel ledit sérum de veau bovin comprend le sérum de veau foetal.

5. Milieu de culture destiné à la croissance in vitro de cellules contenant une quantité de liquide allantoïdien d'un animal gravide ayant un placenta, caractérisé en ce qu'il contient en outre une quantité de sérum de veau bovin mélangée à celui-ci.

6. Milieu selon la revendication 5, ledit sérum de veau bovin comprenant le sérum de veau foetal.

7. Milieu selon les revendications 5 ou 6, dans lequel ledit animal est sélectionné dans le groupe comprenant les brebis, les juments, les vaches et les truies.

**Patentansprüche**

1. Verfahren zum in vitro-Kultivieren von Zellen in einem Medium, welches Allantoisflüssigkeit eines trächtigen Tieres mit einer Placenta enthält, welches das Entnehmen einer Menge Allantoisflüssigkeit von dem Tier, das Mischen einer Menge bovinen Kälberserums mit der Allantoisflüssigkeit, um ein gemischtes Produkt zu bilden, das Überführen des gemischten Produkts in ein in vitro-Gefäß getrennt und entfernt von dem Tier, das Überführen und Kontaktieren der zu kultivierenden Zellen mit dem gemischten Produkt in dem Gefäß, und das Wachsenlassen der Zellen darin umfaßt.

2. Verfahren nach Anspruch 1, bei dem das Tier aus der Gruppe bestehend aus Schafen, Pferden, Kühen und Schweinen ausgewählt ist.

3. Verfahren nach den Ansprüchen 1 oder 2, bei dem die Zellen Hybridomzellen sind.

4. Verfahren nach den Ansprüchen 1 bis 3, bei dem das bovine Kälberserum fetales Kälberserum umfaßt.

5. Kulturmedium zum in vitro-Kultivieren von Zellen, welches eine Menge Allantoisflüssigkeit eines trächtigen Tieres mit einer Placenta enthält, **dadurch gekennzeichnet**, daß es ferner eine Menge bovinen Kälberserums mit derselben vermischt enthält.

6. Medium nach Anspruch 5, bei dem das bovine Kälberserum fetales Kälberserum umfaßt.

7. Medium nach den Ansprüchen 5 oder 6, bei dem das Tier aus der Gruppe bestehend aus Schafen, Pferden, Kühen und Schweinen ausgewählt ist.